# EUROPÄISCHE PATENTSCHRIFT

(11) **EP 4 249 197 B1**
(45) Veröffentlichungstag und Bekanntmachung des Hinweises auf die Patenterteilung: **22.04.2026**
(21) Anmeldenummer: 23160651.8
(22) Anmeldetag: 08.03.2023
(51) Int. Cl.: B29B 7/72, G05D 11/13, G01N 33/44

(54) **VERFAHREN ZUM ERMITTELN EINES MISCHUNGSVERHÄLTNISSES**
METHOD FOR DETERMINING A MIXING RATIO
PROCÉDÉ DE DÉTERMINATION D'UN RAPPORT DE MÉLANGE

(30) Priorität: 24.03.2022 DE 102022001023; 27.12.2022 DE 102022134834
(43) Veröffentlichungstag der Anmeldung: 27.09.2023
(73) Patentinhaber: Deutsches Zentrum für Luft- und Raumfahrt e.V., 53227 Bonn-Oberkassel (DE)
(72) Erfinder: Kühn, Alexandra, 21684 Agathenburg (DE); Grohmann, Yannis, 20249 Hamburg (DE)
(74) Vertreter: Gramm, Lins & Partner Patent- und Rechtsanwälte PartGmbB

(56) Entgegenhaltungen:
- CN-A- 113 484 495
- DE-A1- 102018 130 953
- US-A1- 2015 152 759

## Beschreibung

Die Erfindung betrifft ein Verfahren zum Ermitteln eines Mischungsverhältnisses einer in einem Rohrleitungssystem strömenden, mehrkomponentigen homogenen Materialmischung in Form eines Mehrkomponenten-Epoxidharzsystems, mittels einer die Materialmischung berührende Sensorik, die mindestens ein Sensor mit einer Sensoroberfläche hat, die von der strömenden Materialmischung zur Ermittlung des Mischungsverhältnisses kontaktiert wird.

Die Erfindung betrifft außerdem ein Verfahren zur Herstellung eines Faserverbundbauteils, bei dem ein Fasermaterial mit der Mehrkomponenten-Matrixmaterialmischung infundiert und die infundierte Mehrkomponenten- Matrixmaterialmischung ausgehärtet wird.

Aufgrund der gewichtsspezifischen Festigkeit und Steifigkeit sind Faserverbundwerkstoffe aus der Luft- und Raumfahrt sowie aus anderen gewichtskritischen Bereichen kaum mehr wegzudenken. Faserverbundwerkstoffe weisen dabei hauptsächlich zwei wesentliche Bestandteile auf, nämlich zum einen ein Fasermaterial und zum anderen ein das Fasermaterial einbettendes Matrixmaterial. Bei der Herstellung von Faserverbund-Bauteilen aus einem solchen Faserverbundwerkstoff wird dabei in der Regel das Fasermaterial in die entsprechende spätere Bauteilform gebracht und anschließend dann das Matrixmaterial ausgehärtet. Das Aushärten geschieht in den allermeisten Fällen durch Temperatur- und gegebenenfalls Druckbeaufschlagung, wobei durch das Aushärten die lasttragenden Fasern des Fasermaterials in ihre vorbestimmte Richtung gezwungen werden und dabei zusammen mit dem ausgehärteten Matrixmaterial eine integrale Einheit zur Lastabtragung bilden.

Das Matrixmaterial, welches das Fasermaterial des Faserverbundwerkstoffes entsprechend einbettet, kann dabei bereits in dem Fasermaterial enthalten sein (sogenannte Prepregs) oder später in eine sogenannte Faserpreform, die aus trockenen Fasermaterialien aufgebaut wurde, in einem Infusionsprozess infundiert werden. Eine Faserpreform stellt dabei eine Art Vorbauteil (Preform) dar, das aus dem Fasermaterial des Faserverbundwerkstoffes gebildet und dabei zumindest teilweise die spätere Bauteilform des herzustellenden Faserverbundbauteils enthält. Durch das Aushärten des Matrixmaterials, welches in dem Fasermaterial der Faserpreform eingebettet ist, kann so das Faserverbundbauteil hergestellt werden. Die Faserpreform kann demzufolge sowohl aus trockenem Fasermaterial als auch aus vorimprägnierten Fasermaterialien eines Faserverbundwerkstoffes hergestellt werden. Als Faserverbundwerkstoffe kommen dabei unterschieden nach der Art des Fasermaterials insbesondere CFK und GFK zum Einsatz.

Es besteht die Bestrebung, bei der Verwendung von trockenen Fasermaterialien ein Matrixmaterial einzusetzen, das aus mehreren Komponenten vor Ort kurz vor dem Infundieren des Matrixmaterials in das Fasermaterial zusammengesetzt wird. Hierfür eignen sich Mehrkomponenten-Epoxidharzsysteme. Der Vorteil gegenüber Einkomponenten-Harzsystemen besteht darin, dass das Material in einzelne Komponenten aufgeteilt wird, insbesondere in eine Harzkomponente und in eine Härterkomponente. Hierdurch wird die Vernetzung des Matrixmaterials stark verzögert oder sogar verhindert. Die Komponenten können so unter erheblich verringerten Sicherheitseinschränkungen transportiert und gelagert werden. Die Lagerung kann bei erhöhter Temperatur stattfinden und die Haltbarkeit der einzelnen Komponenten verlängert sich.

Kurz vor dem Infundieren des Matrixmaterials in das Fasermaterial des Bauteils werden die einzelnen Komponenten des Matrixsystems in einem vorgegebenen Mischungsverhältnis zusammengeführt und homogen vermengt. Anschließend wird die so hergestellte Materialmischung in das Fasermaterial infundiert. Das Zusammenführen und Vermengen der einzelnen Komponenten, um das Meerkomponenten-Matrixsystem herzustellen, kann dabei inline im Prozess erfolgen, d. h. dass die zusammengeführten Komponenten durch ein Rohrleitungssystem oder Schlauchsystem sofort in das Bauteil infundiert werden.

Eine wesentliche Voraussetzung bei der Verwendung eines mehr Komponenten-Matrixsystems besteht darin, dass das richtige Mischungsverhältnis möglichst direkt im Prozess nachweisbar ist. Nur so kann sichergestellt werden, dass das hergestellte Bauteil mit dem spezifizierten Matrixsystem hergestellt wurde. Dies ist insoweit deshalb notwendig, da das Mischungsverhältnis im Prozess selber während des Infundierens des Matrixsystems eingestellt wird.

Bei der Analyse des Mischungsverhältnisses von Mehrkomponenten-Matrixsystem kommen normalerweise berührende Sensorik in zum Einsatz, deren Sensoren einen direkten Kontakt zum strömenden Medium des Matrixsystems erfordern. Es hat sich jedoch gezeigt, dass sich bei diesen Sensoren ein Materialfilm (Harzfilm) direkt auf dem Sensor bildet, der von dem nachströmenden Material (Harz) weggedrückt werden muss. Wenn die Viskosität des nachströmenden Materials höher ist, lässt sich die Änderung im Mischungsverhältnis gut von der jeweiligen Sensorik abbilden. Ist die Viskosität jedoch geringer, kann die Änderung des Mischungsverhältnisses von der Sensorik schlecht bis gar nicht erfasst werden, weil das niederviskose, nachströmenden Material den Materialfilm, der sich direkt auf dem Sensor gebildet hat, nicht wegdrücken kann.

Es entsteht eine Phase, die vom Sensor zwar korrekt analysiert wird, jedoch nicht dem (Harze) auch von der Temperatur abhängig ist, treten entsprechende Probleme auf wenn sich die den Sensor berührende Schicht im Vergleich zum nachströmenden Medium abkühlt. Zur Behebung des Problems müsste ein Spülvorgang stattfinden, bevor die Änderung des Mischungsverhältnisses zu einer Verringerung der Viskosität der Harzmischung führt.

In der Praxis ist eine solche Änderung des Mischungsverhältnisses im Prozess nicht planbar. Vielmehr dient die Mischungsanalyse dazu, solche unbeabsichtigten Änderungen im Rahmen des Qualitätsmanagements zu erfassen. Aktuell kann es also dazu kommen, dass eine Störung im Prozess nicht von der Analytik erfasst wird und ein Bauteil mit fehlerhaftem Mischungsverhältnis nicht als Ausschussteil erkannt wird. Da vom Mischungsverhältnis auch mechanische und thermische Kennwerte abhängig sind, kann es hier zu großen Sicherheitseinbußen kommen. Als Konsequenz daraus kann eine Analytik, bei der die benannten Probleme auftreten, in der Produktion nicht eingesetzt werden und somit auch keine Mehrkomponenten-Epoxidharzsysteme, deren Einzelkomponenten unterschiedliche Viskositäten aufweisen.

Die US 2015/0152759 A1 offenbart ein Wasserabscheidungssystem für Öltanks, um das sich in dem Öl gesammelte Wasser abscheiden zu können. Dabei ist ein Sensor vorgesehen, um Wasser im Öl innerhalb einer Leitung feststellen zu können.

Ein ähnliches Vorgehen offenbart auch die CN113484495A, bei der Partikel in einem Dieselfahrzeug in der Kraftstoffzufuhr detektiert werden sollen. Auch hier sind verschiedene Sensoren vorgesehen, die mithilfe einer mechanischen Reinigung gereinigt werden sollen.

Aus der DE 10 2018 130 953 A1 ist ein Verfahren und eine Vorrichtung zum Ermitteln eines Mischungsverhältnisses einer Materialmischung bekannt, die aus zwei oder mehr Komponenten gebildet wird. Zur Ermittlung des Mischungsverhältnisses wird dabei ein dielektrischer Kennwert ermittelt.

Es ist daher Aufgabe der vorliegenden Erfindung ein verbessertes Verfahren zur Mischungsanalyse bei der Verwendung von Mehrkomponenten-Matrixsystem anzugeben, ohne dass hierfür ein Prozess unterbrochen werden muss.

Die Aufgabe wird mit dem Verfahren zum Ermitteln eines Mischungsverhältnisses einer strömenden, mehrkomponentigen Materialmischung gemäß Anspruch 1 erfindungsgemäß gelöst. Vorteilhafte Ausgestaltungen der Erfindung finden sich in den entsprechenden Unteransprüchen.

Gemäß Anspruch 1 wird ein Verfahren zum Ermitteln eines Mischungsverhältnisses einer strömenden, mehrkomponentigen homogenen Materialmischung in Form eines Mehrkomponenten-Epoxidharzsystems vorgeschlagen, die innerhalb eines Rohleitungssystems (beispielsweise ein Rohr oder ein Schlauch) strömt. Für die Ermittlung eines Mischungsverhältnisses wird hierfür eine berührende Sensorik vorgesehen, die mit einem Sensor mit der in dem Rohleitungssystem strömenden Materialmischung in Wirkverbindung steht. Der Sensor weist hierfür eine Sensoroberfläche auf, die von der strömenden Materialmischung zur Ermittlung des Mischungsverhältnisses kontaktiert wird.

Die strömende Materialmischung wurde dabei aus zwei oder mehr Einzelkomponenten gemischt und liegt so als mehrkomponentige Materialmischung vor.

Erfindungsgemäß ist in einem ersten Aspekt vorgesehen, dass mittels eines mechanischen Reinigers die an der Sensoroberfläche haftende Materialmischung durch eine Relativbewegung zwischen Sensor und/oder Sensoroberfläche und Reiniger entfernt oder reduziert wird.

Es wird somit vorgeschlagen, dass mithilfe eines mechanischen Reinigers, der mit der Sensoroberfläche des Sensors mechanisch in Wirkverbindung steht, die Sensoroberfläche gereinigt wird, indem an der Sensoroberfläche haftende Reste der Materialmischung durch den mechanischen Reiniger aufgrund einer Relativbewegung zwischen Sensoroberfläche und Reiniger entfernt oder reduziert werden.

Hierdurch wird das Ziel erreicht, dass die Sensoroberfläche regelmäßig mit derjenigen Materialmischung in Kontakt kommt, die tatsächlich auch an dem Sensor vorbeiströmt. Es wird somit verhindert, dass Reste einer Materialmischung auf der Sensoroberfläche verbleiben, wodurch die Gefahr besteht, dass der Sensor das aktuelle Mischungsverhältnis der Materialmischung nicht mehr richtig erfassen kann. Durch das Entfernen der an der Sensoroberfläche haftenden Materialmischung durch den mechanischen Reiniger kommt der Sensor regelmäßig mit der aktuellen Materialmischung, die an dem Sensor vorbeiströmt, in Kontakt und kann so das aktuelle Mischungsverhältnis ermitteln, sodass plötzlich auftretende Änderungen im Mischungsverhältnis oder nicht zu erwartenden Veränderungen im Mischungsverhältnis frühzeitig erkannt werden.

Die Relativbewegung zwischen der Sensoroberfläche und dem mechanischen Reiniger kann dabei derart erfolgen, dass der Sensor mit seiner Sensoroberfläche fest verbaut ist und der mechanische Reiniger in Art eines Wischers über die Sensoroberfläche streicht und dabei an der Sensoroberfläche haftende Reste der Materialmischung entfernt. Denkbar ist aber auch, dass die Sensoroberfläche und/oder der Sensor beweglich verbaut sind und sich gegenüber einem fest verbauten mechanischen Reiniger bewegen. Auch hierbei werden Reste der Materialmischung von der Sensoroberfläche entfernt.

Der mechanische Reiniger kann dabei so bereitgestellt und ausgebildet sein, dass der Reinigungskontakt zwischen der Sensoroberfläche und dem Reiniger flexibel ist, beispielsweise durch einen flexiblen Reiniger aus einem flexiblen Material wie Gummi, Kautschuk oder Silikon. Denkbar ist aber auch, dass die Sensoroberfläche bzw. der Sensor flexibel ausgebildet ist, um einen flexiblen Reinigungskontakt zu ermöglichen. Hierfür kann der Sensor beispielsweise eine flexible Lagerung aufweisen, indem der Sensor auf einer flexiblen Oberfläche aufgebracht ist.

Der Reiniger kann ähnlich einem Gummiwischer ausgebildet sein. Denkbar ist aber auch ein fester Metall-Stempel, der durch Verdrängung die anhaftende Materialmischung entfernt bzw. verdrängt.

Ein solcher Sensor kann bspw. ein Sensor sein der basierend auf einer Dielektrizitätsmessung (DEA) das Mischungsverhältnis ermittelt. Aber auch Sensoren basierend auf Ultraschallmessung, Refraktometrie, Near Field Infrared (NIR), Rheometrie und/oder einer bildgebenden Sensorik sind zur Ermittlung des Mischungsverhältnisses geeignet.

Erfindungsgemäß ist in einem zweiten Aspekt vorgesehen, dass der Sensor mit der Sensoroberfläche zum Ermitteln des Mischungsverhältnisses derart bereitgestellt wird, dass die Sensoroberfläche einer Antihaftbeschichtung aufweist.

Hierdurch wird das Ziel erreicht, dass die an der Sensoroberfläche entlang strömende Materialmischung nicht an der Sensoroberfläche haftet, wenn die mehrkomponentiege Materialmischung einen gewissen Viskositätsschwellwert unterschreitet. Hierdurch wird sichergestellt, dass die Sensoroberfläche immer frei ist von Rückständen der Materialmischung, da diese nicht an der Antihaftbeschichtung der Sensoroberfläche haften.

Eine solche Antihafteigenschaft kann beispielsweise durch eine entsprechende Oberflächenstruktur realisiert werden, die einen Lotuseffekt erzeugt. Aber auch eine Beschichtung mit einem geeigneten Antihaftmittel ist hierbei denkbar.

Der erste und der zweite Aspekt Erfindung können dabei als Alternativen vorliegen oder auch gemeinsam in Kombination.

Gemäß einer Ausführungsform ist vorgesehen, dass die an der Sensoroberfläche haftende Materialmischung mittels eines stabförmigen Reinigers, mittels einer Gummilippe als Reiniger, mittels eines Schwamms als Reiniger und/oder mittels eines Schabers als Reiniger entfernt wird. Der Schaber kann dabei aus einem biegesteifen Material bestehen, bspw. Metall.

Ein solcher stabförmiger Reiniger erstreckt sich dabei vorzugsweise über die gesamte Ausdehnung der Sensoroberfläche und kann somit durch eine einzige Relativbewegung die gesamte Sensoroberfläche reinigen.

Gemäß einer Ausführungsform ist vorgesehen, dass der Reiniger zum Entfernen der an der Sensoroberfläche haftenden Materialmischung translatorisch und/oder rotatorisch und/oder vibrierend bewegt wird.

In Kombination mit dem stabförmigen Reiniger es ist demnach vorteilhaft, wenn der stabförmige Reiniger translatorischen über die gesamte Sensoroberfläche bewegt wird. Denkbar ist aber auch, dass der stabförmige Reiniger sich um seine eigene Achse rotatorisch dreht und dabei über die Sensoroberfläche streicht. Auch in Kombination mit einer Vibration und beispielsweise Zusammenhang mit einer translatorischen Bewegung kann die Reinigung der Sensoroberfläche erfolgen.

Erfindungsgemäß ist vorgesehen, dass die mehrkomponentige Materialmischung ein Mehrkomponenten-Epoxidharzsystem ist.

Ein solches Mehrkomponenten-Epoxidharzsystem kann dabei beispielsweise als Materialmischung aus den beiden Einzelkomponenten Epoxidharz und Härter gebildet werden.

Gemäß einer Ausführungsform ist vorgesehen, dass die mehrkomponentige Materialmischung bei der gewünschten Prozesstemperatur eine Viskosität von weniger als 1000 mPa*s, vorzugsweise weniger als 500 mPa*s, besonders vorzugsweise weniger als 100 mPa*s. Denkbar ist aber auch, dass die mehrkomponentige Materialmischung bei der gewünschten Prozesstemperatur eine deutlich geringere Viskosität von weniger als 25 mPa*s, vorzugsweise weniger als 10 mPa*s, besonders vorzugsweise weniger als 3 mPa*s. Es kann hierbei besonders bevorzugt sein, wenn die mehrkomponentige Materialmischung eine Viskosität bei Prozesstemperatur aufweist, die annähernd der Viskosität von Wasser bei einer Temperatur von 5° oder höher aufweist.

So kann durch eine niedrige Viskosität in Verbindung mit einer Antihaftbeschichtung der Sensoroberfläche und/oder dem mechanischen Reiniger erreicht werden, dass Rückstände der Materialmischung nicht haften bleiben und sich darüber hinaus sehr leicht von der Sensoroberfläche durch den mechanischen Reiniger entfernen lassen.

Gemäß einer Ausführungsform ist vorgesehen, dass in Abhängigkeit von dem ermittelten Mischungsverhältnis ein Wegeventil innerhalb des Rohrleitungssystems derart gesteuert wird, dass bei einem unzureichenden Mischungsverhältnis die Materialmischung als Ausschuss aus dem Rohrleitungssystem abgeleitet wird.

Das Wegeventil ist dabei in Fließrichtung der Materialmischung nach der die Materialmischung berührende Sensorik in dem Rohrleitungssystem vorgesehen, sodass mithilfe einer mit der Sensorik in Verbindung stehender Steuereinrichtung auf das unzureichende Mischungsverhältnis so reagiert werden kann, dass das Wegeventil in eine Position gebracht wird, in der die Materialmischung als Ausschuss aus dem Rohrleitungssystem abgeleitet wird. Die Materialmischung wird dann nicht mehr für den Produktivbetrieb verwendet, beispielsweise nicht mehr in ein Fasermaterial eines Faserverbundbauteils eingeleitet. Wird hingegen ein ausreichendes Mischungsverhältnis festgestellt, welches im Produktivbetrieb verwendet werden kann, so wird das Wegeventilen eine Position gebracht, in der die Materialmischung für den Produktivbetrieb verwendet werden kann, beispielsweise derart, dass die Materialmischung in ein Fasermaterial infundiert wird.

Hierdurch wird sichergestellt, dass bei einem schlechten Mischungsverhältnis die Materialmischung nicht das gesamte Bauteil verunreinigt. Hierdurch lässt sich der Ausschuss durch schlechte Materialmischungen verringern und somit Kosten einsparen. Die Aufgabe wird im Übrigen auch mit dem Verfahren zur Herstellung eines Faserverbundbauteils gemäß Anspruch 7 erfindungsgemäß gelöst, bei dem ein Fasermaterial mit einer Mehrkomponenten-Matrixmaterialmischung infundiert und die infundierte Mehrkomponenten- Matrixmaterialmischung ausgehärtet wird. Das Verfahren umfasst dabei die folgenden Schritte:
- Bereitstellen der einzelnen Komponenten der Mehrkomponenten- Matrixmaterialmischung,
- Zusammenführen und Vermengen der einzelnen Komponenten, um die Mehrkomponenten- Matrixmaterialmischung zu erhalten, und
- Infundieren der Mehrkomponenten- Matrixmaterialmischung in das Fasermaterial,
- Ermitteln eines Mischungsverhältnisses der strömenden Mehrkomponenten- Matrixmaterialmischung gemäß dem vorstehend beschriebenen Verfahren.

Die einzelnen Komponenten werden dabei in einen nicht gemischten Formkern bereitgestellt, d. h. als Einzelkomponenten. Vorzugsweise werden die einzelnen Komponenten dann während des Infundierens in das Fasermaterial zusammengeführt und vermengt, sodass das Vermischen der Einzelkomponenten inline im Infusionsprozess erfolgt.

Gemäß einer Ausführungsform ist vorgesehen, das Infundieren der Mehrkomponenten- Matrixmaterialmischung in Abhängigkeit von dem ermittelten Mischungsverhältnis gesteuert wird.

So kann der Prozess beispielsweise gestoppt werden, wenn festgestellt wird, dass das Mischungsverhältnis nicht innerhalb des vorgegebenen Toleranzbereiches liegt. So ist es denkbar, dass mithilfe eines Ventils das Infundieren gestoppt wird, ist das Mischungsverhältnis wieder innerhalb des vorgegebenen Toleranzbereiches liegt.

Die Erfindung wird anhand der beigefügten Figuren beispielhaft näher erläutert. Es zeigen:
- Figur 1: schematische Darstellung einer Fertigungsanlage Herstellung eines Faserverbundbauteils;
- Figur 2: schematische Darstellung eines Sensors mit Reiniger.

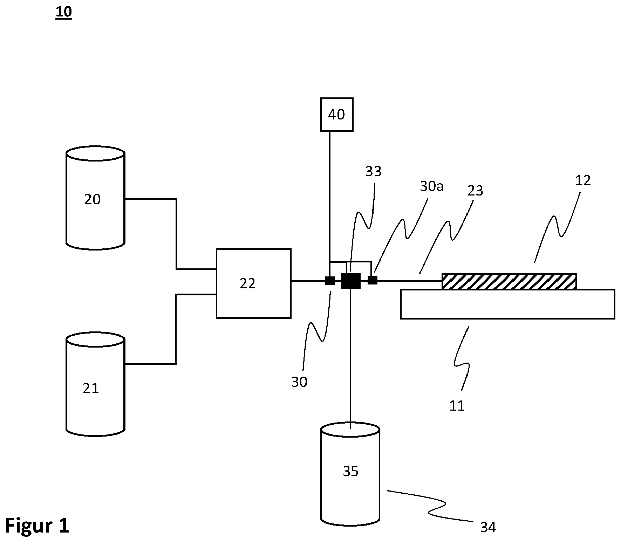

Figur 1 zeigt eine Fertigungsanlage 10, mit der ein Faserverbundbauteil hergestellt werden kann. Hierfür wird ein Formwerkzeug 11 bereitgestellt, auf das ein Fasermaterial 12 zur Herstellung einer Faserpreform abgelegt wird. Das trockene Fasermaterial 12 wird nach dem Eindringen des Fasermaterials 12 in das Formwerkzeug 11 mit einem Mehrkomponenten-Matrixsystem (bspw. ein Mehrkomponenten-Epoxidharzsystem) infundiert, um das Fasermaterial 12 vollständig zu Tränken. Anschließend wird das Matrixsystem ausgehärtet, um das Bauteil herzustellen.

Das Mehrkomponenten-Matrixsystem wird dabei während des Infundierens in das Fasermaterial aus zwei Einzelkomponenten gemischt, die getrennt als Vernetzungsmaterial 20 (Harzsystem) und als Härtermaterial 21 bereitgestellt werden. Mithilfe einer Mischvorrichtung 22 werden das Vernetzungsmaterial 20 und das Härtermaterial 21 zu einem gemeinsamen Mehrkomponenten-Matrixsystem vermengt, dass dann das Fasermaterial 12 infundiert wird.

Das so gemischte Mehrkomponenten-Matrixsystem wird nun über ein Rohrleitungssystem 23 dem Fasermaterial 12 zugeführt, umso das Fasermaterial 12 mit dem Mehrkomponenten-Matrixsystem zu tränken.

In dem Rohrleitungssystem 23 ist dabei ein erster Sensor 30 angeordnet, der ausgebildet ist, das Mischungsverhältnis des Mehrkomponenten-Matrixsystem zu ermitteln. Der Sensor 30 steht dabei mit einer Steuereinheit 40 in Verbindung, die zur Steuerung des Anlagenprozesses der Fertigungsanlage 10 ausgebildet ist.

Mithilfe des Sensors 30 wird dabei kontinuierlich das Mischungsverhältnis des Mehrkomponenten-Matrixsystem überwacht, welches in Richtung des Fasermaterials 12 fließt.

Wird dabei ein Mischungsverhältnis festgestellt, dass außerhalb des Toleranzbereiches liegt, so kann die Fertigungsanlagen 10 eingerichtet sein, den Materialfluss in Richtung Fasermaterial 12 zu unterbrechen oder abzutrennen, um zu verhindern, dass das Fasermaterial 12 mit einem Mehrkomponenten-Matrixsystem infundiert wird, welches ein falsches Mischungsverhältnis hat. Wenn der Fehler behoben ist und das Mischungsverhältnis wieder innerhalb des Toleranzbereiches liegt, kann der Materialfluss wieder aufgenommen werden bzw. das Rohrleitungssystem 30 wieder so eingestellt werden, dass ein Materialfluss in das Fasermaterial 12 möglich ist.

In Figur 1 ist eine vorteilhafte Weiterbildung gezeigt, bei der ein zweiter Sensor 30a in dem Rohrleitungssystem 23 vorgesehen ist, der ebenfalls mit der Steuereinheit 40 in Verbindung stehen und zum Erfassen des Mischungsverhältnisses der Materialmischung ausgebildet ist. Zwischen dem ersten Sensor 30 und dem zweiten Sensor 30a ist in dem Rohrleitungssystem 23 ein Wegeventil 33 vorgesehen, dass eingerichtet ist, entweder eine Durchleitung von der Mischvorrichtung 22 in das Fasermaterial 12 oder eine Ableitung der Materialmischung aus dem Rohrleitungssystem als Ausschuss einzustellen.

Wird von dem ersten Sensor 30 ein Mischungsverhältnis detektiert, welches von der Steuereinheit 40 als nicht akzeptabel eingestuft wird, kann der gesamte Infusionsprozess unterbrochen werden, wobei das Wegeventil so eingestellt wird, dass die Materialmischung als Ausschuss aus dem Rohrleitungssystem abgeleitet wird. Der zweite Sensor 30a, der nach dem Wegeventil in dem Rohrleitungssystem angeordnet ist, überwacht dabei, dass keine unzureichende Materialmischung in das Bauteil gelangt. Erst wenn durch den ersten Sensor 30 eine akzeptable Materialmischung detektiert wird, kann das Wegeventil wieder eine Position gebracht werden, in der eine Durchleitung in das Fasermaterial zum Infundieren möglich ist.

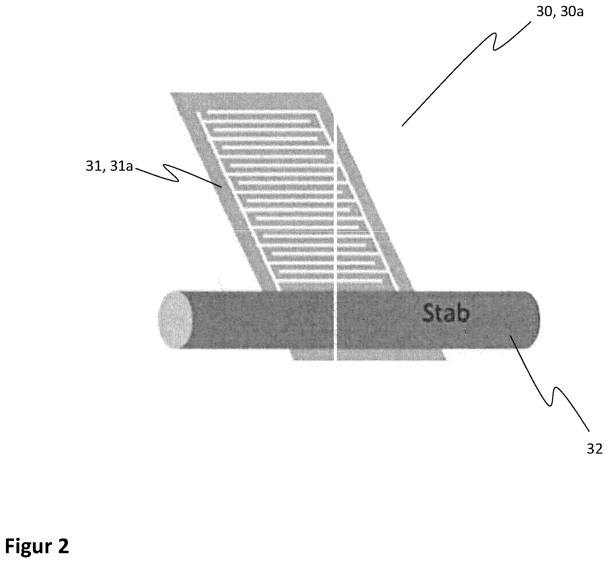

Figur 2 zeigt in einer schematisch vereinfachten Darstellung einen Sensor 30, 30a, der eine Sensoroberfläche 31, 31a hat, die mit dem Mehrkomponenten-Matrixsystem in Kontakt kommt, wenn das Mehrkomponenten-Matrixsystem in das Fasermaterial 12 infundiert wird oder allgemeiner, wenn das Mehrkomponenten-Matrixsystem in einem Fluidkanal strömt.

Um die Sensoroberfläche 31, 31a von möglichen Resten des Mehrkomponenten-Matrixsystems zu befreien, ist ein stabförmiger Reiniger 32 vorgesehen, der so ausgebildet ist, dass er entlang der Sensoroberfläche 31, 31a verfahrbar ist und so Reste der Materialmischung entfernen kann. Hierfür kann der Reiniger 32 aus einem flexiblen Material gebildet sein, beispielsweise um Gummi.

Dabei kann vorgesehen sein, dass die Sensoroberfläche 31, 31a mit einer Antihaftbeschichtung beschichtet ist, umso unter Ausnutzung des Lotuseffektes eine mögliche Anhaftung weitgehend zu vermeiden.

### Bezugszeichenliste

10 Fertigungsanlagen
11 Formwerkzeug
12 Fasermaterial
20 erste Komponente/Vernetzungsmaterial/Harz
21 zweite Komponente/Härter
22 Mischvorrichtung
23 Rohrleitungssystem
30 Sensor
31 Sensoroberfläche
32 mechanischer Reiniger
33 Wegeventil
34 Spülbehälter
35 Spülmittel
40 Steuereinheit

## Patentansprüche

1. Verfahren zum Ermitteln eines Mischungsverhältnisses einer in einem Rohrleitungssystem (23) strömenden, mehrkomponentigen homogenen Materialmischung in Form eines Mehrkomponenten-Epoxidharzsystems mittels einer die homogene Materialmischung berührende Sensorik, die mindestens einen Sensor (30) mit einer Sensoroberfläche (31) hat, die von der strömenden Materialmischung zur Ermittlung des Mischungsverhältnisses kontaktiert wird, **dadurch gekennzeichnet, dass**
- mittels eines mechanischen Reinigers (32) die an der Sensoroberfläche (31) haftende Materialmischung durch eine Relativbewegung zwischen Sensor (30) und/oder Sensoroberfläche (31) und Reiniger (32) entfernt oder reduziert wird, und/oder
- dass ein Sensor (30) mit einer Sensoroberfläche (31) bereitgestellt wird, die eine Antihaftbeschichtung aufweist.

2. Verfahren nach Anspruch 1, **dadurch gekennzeichnet, dass** die an der Sensoroberfläche (31) haftende Materialmischung mittels eines stabförmigen Reinigers (32), mittels einer Gummilippe als Reiniger, mittels eines Schwamms als Reiniger und/oder mittels eines Schabers als Reiniger entfernt wird.

3. Verfahren nach Anspruch 1 oder 2, **dadurch gekennzeichnet, dass** die Relativbewegung durch ein Bewegen des Sensors (30) und/oder durch ein Bewegen des Reinigers (32) erfolgt.

4. Verfahren nach einem der vorhergehenden Ansprüche, **dadurch gekennzeichnet, dass** der Reiniger (32) zum Entfernen der an der Sensoroberfläche (31) haftenden Materialmischung translatorisch und/oder rotatorisch und/oder vibrierend bewegt wird.

5. Verfahren nach einem der vorhergehenden Ansprüche, **dadurch gekennzeichnet, dass** die mehrkomponentige Materialmischung bei der gewünschten Prozesstemperatur eine Viskosität von weniger als 1000 mPa*s, vorzugsweise weniger als 500 mPa*s, besonders vorzugsweise weniger als 100 mPa*s aufweist.

6. Verfahren nach einem der vorhergehenden Ansprüche, **dadurch gekennzeichnet, dass** in Abhängigkeit von dem ermittelten Mischungsverhältnis ein Wegeventil innerhalb des Rohrleitungssystems derart gesteuert wird, dass bei einem unzureichenden Mischungsverhältnis die Materialmischung als Ausschuss aus dem Rohrleitungssystem abgeleitet wird.

7. Verfahren zur Herstellung eines Faserverbundbauteils, bei dem ein Fasermaterial (12) mit einer Mehrkomponenten-Matrixmaterialmischung infundiert und die infundierte Mehrkomponenten-Matrixmaterialmischung ausgehärtet wird, mit
- Bereitstellen der einzelnen Komponenten (20, 21) der Mehrkomponenten-Matrixmaterialmischung,
- Zusammenführen und Vermengen der einzelnen Komponenten (20, 21), um die Mehrkomponenten-Matrixmaterialmischung zu erhalten, und
- Infundieren der Mehrkomponenten-Matrixmaterialmischung in das Fasermaterial (12),
**gekennzeichnet durch**
- Ermitteln eines Mischungsverhältnisses der strömenden Mehrkomponenten- Matrixmaterialmischung gemäß dem Verfahren nach einem der Ansprüche 1 bis 6.

8. Verfahren nach Anspruch 7, **dadurch gekennzeichnet, dass** das Infundieren der Mehrkomponenten- Matrixmaterialmischung in Abhängigkeit von dem ermittelten Mischungsverhältnis gesteuert wird.

9. Verfahren nach Anspruch 7 oder 8, **dadurch gekennzeichnet, dass** in Abhängigkeit von dem ermittelten Mischungsverhältnis ein Wegeventil innerhalb des Rohrleitungssystems derart gesteuert wird, dass bei einem unzureichenden Mischungsverhältnis die Mehrkomponenten-Matrixmaterialmischung nicht in das Fasermaterial infundiert wird.

## Claims

1. Method for determining a mixing ratio of a multi-component homogeneous material mixture flowing in a pipe system (23) in the form of a multi-component epoxy resin system by means of a sensor system contacting the homogeneous material mixture, which has at least one sensor (30) with a sensor surface (31) that is contacted by the flowing material mixture for determining the mixing ratio, **characterized in that**
- a mechanical cleaner (32) is used to remove or reduce the material mixture adhering to the sensor surface (31) by means of a relative movement between the sensor (30) and/or sensor surface (31) and the cleaner (32), and/or
- a sensor (30) is provided with a sensor surface (31) that has a non-stick coating.

2. Method according to claim 1, **characterized in that** the mixture of materials adhering to the sensor surface (31) is removed by means of a rod-shaped cleaner (32), by means of a rubber lip as a cleaner, by means of a sponge as a cleaner, and/or by means of a scraper as a cleaner.

3. Method according to claim 1 or 2, **characterized in that** the relative movement is effected by moving the sensor (30) and/or by moving the cleaner (32).

4. Method according to one of the preceding claims, **characterized in that** the cleaner (32) is moved in a translatory and/or rotary and/or vibrating manner to remove the mixture of materials adhering to the sensor surface (31).

5. Method according to one of the preceding claims, **characterized in that** the multi-component material mixture has a viscosity of less than 1000 mPa*s, preferably less than 500 mPa*s, particularly preferably less than 100 mPa*s at the desired process temperature.

6. Method according to one of the preceding claims, **characterized in that**, depending on the determined mixing ratio, a directional control valve within the pipe system is controlled in such a way that, if the mixing ratio is insufficient, the material mixture is discharged from the pipe system as waste.

7. Method for producing a fiber composite component, in which a fiber material (12) is infused with a multi-component matrix material mixture and the infused multi-component matrix material mixture is cured, comprising
- providing the separate components (20, 21) of the multi-component matrix material mixture,
- combining and mixing the separate components (20, 21) to obtain the multi-component matrix material mixture, and
- infusing the multi-component matrix material mixture into the fiber material (12),
**characterized by**
- determining a mixing ratio of the flowing multi-component matrix material mixture according to the method according to one of claims 1 to 6.

8. Method according to claim 7, **characterized in that** the infusion of the multi-component matrix material mixture is controlled depending on the determined mixing ratio.

9. Method according to claim 7 or 8, **characterized in that**, depending on the determined mixing ratio, a directional control valve within the pipe system is controlled in such a way that, if the mixing ratio is insufficient, the multi-component matrix material mixture is not infused into the fiber material.

## Revendications

1. Procédé pour déterminer le rapport de mélange d'un mélange de matériaux homogène à plusieurs composants sous la forme d'un système de résine époxy à plusieurs composants, s'écoulant dans un système de tuyauterie (23), à l'aide d'un ensemble de capteurs qui est en contact avec le mélange de matériaux homogène et qui comporte au moins un capteur (30) ayant une surface de capteur (31) qui est en contact avec le mélange de matériaux en écoulement afin de déterminer le rapport de mélange,
**caractérisé en ce que**
- un nettoyeur mécanique (32) permet d'éliminer ou de réduire le mélange de matériaux adhérant à la surface de capteur (31) par un mouvement relatif entre le capteur (30) et/ou la surface de capteur (31) et le nettoyeur (32), et/ou
- **en ce qu'**il est prévu un capteur (30) ayant une surface de capteur (31) qui présente un revêtement antiadhésif.

2. Procédé selon la revendication 1,
**caractérisé en ce que** le mélange de matériaux adhérant à la surface de capteur (31) est éliminé à l'aide d'un nettoyeur en forme de tige (32), à l'aide d'une lèvre en caoutchouc faisant office de nettoyeur, à l'aide d'une éponge faisant office de nettoyeur et/ou à l'aide d'un grattoir faisant office de nettoyeur.

3. Procédé selon la revendication 1 ou 2,
**caractérisé en ce que** le mouvement relatif se fait par un mouvement du capteur (30) et/ou par un mouvement du nettoyeur (32).

4. Procédé selon l'une des revendications précédentes,
**caractérisé en ce que** le nettoyeur (32) est déplacé en translation et/ou en rotation et/ou par vibrations pour éliminer le mélange de matériaux adhérant à la surface de capteur (31).

5. Procédé selon l'une des revendications précédentes,
**caractérisé en ce que** le mélange de matériaux à plusieurs composants présente, à la température de traitement souhaitée, une viscosité inférieure à 1000 mPa*s, de préférence inférieure à 500 mPa*s, et de manière particulièrement préférée inférieure à 100 mPa*s.

6. Procédé selon l'une des revendications précédentes,
**caractérisé en ce que**, en fonction du rapport de mélange déterminé, une vanne de distribution à l'intérieur du système de tuyauterie est commandée de telle sorte que, si le rapport de mélange est insuffisant, le mélange de matériaux est évacué du système de tuyauterie en tant que rebut.

7. Procédé de fabrication d'un composant en matériau composite renforcé de fibres, dans lequel un matériau fibreux (12) est imprégné d'un mélange de matériaux matriciel à plusieurs composants, et le mélange de matériaux matriciel à plusieurs composants imprégné est durci, consistant à
- fournir les composants individuels (20, 21) du mélange de matériaux matriciel à plusieurs composants,
- réunir et mélanger les composants individuels (20, 21) pour obtenir le mélange de matériaux matriciel à plusieurs composants, et
- imprégner le mélange de matériaux matriciel à plusieurs composants dans le matériau fibreux (12),
**caractérisé par**
- la détermination d'un rapport de mélange du mélange de matériaux matriciel à plusieurs composants en écoulement par le procédé selon l'une des revendications 1 à 6.

8. Procédé selon la revendication 7,
**caractérisé en ce que** l'imprégnation du mélange de matériaux matriciel à plusieurs composants est commandée en fonction du rapport de mélange déterminé.

9. Procédé selon la revendication 7 ou 8,
**caractérisé en ce qu'**en fonction du rapport de mélange déterminé, une vanne de distribution à l'intérieur du système de tuyauterie est commandée de telle sorte que, si le rapport de mélange est insuffisant, le mélange de matériaux matriciel à plusieurs composants n'est pas imprégné dans le matériau fibreux.
